# EUROPEAN PATENT APPLICATION

(11) **EP 4 834 321 A1**
(43) Date of publication of application: **04.11.2026**
(21) Application number: 24913285.3
(22) Date of filing: 23.09.2024
(51) Int. Cl.: A61K 8/9789, A61Q 19/00, A61Q 19/02, A61Q 19/08, A61K 36/82, A61P 29/00, A61P 17/00, A61P 17/02

(54) **COMPOSITION COMPRISING CAMELLIA JAPONICA CALLUS-DERIVED EXOSOME AS ACTIVE INGREDIENT FOR IMPROVING SKIN CONDITION**

(30) Priority: 29.12.2023 KR 20230195863
(71) Applicant: GFC Life Science Co., Ltd., Hwaseong-si, Gyeonggi-do 18471 (KR)
(72) Inventor: KANG, Hee Cheol, Hwaseong-si Gyeonggi-do 18471 (KR); KIM, Mi Jung, Seoul 05402 (KR); KIM, Ji Yeoung, Hwaseong-si Gyeonggi-do 18471 (KR)
(74) Representative: Ter Meer und Partner mbB
(86) International application number: PCT/KR2024/014255
(87) International publication number: WO 2025/143444

(57) **Abstract**

Proposed is a cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient.

The *Camellia japonica* callus-derived exosomes provided by the present disclosure can be advantageously used in cosmetic compositions for anti-inflammation, antioxidation, whitening, wrinkle reduction, hydration, skin regeneration, skin barrier improvement, atopic dermatitis alleviation, and skin soothing or in pharmaceutical compositions for anti-inflammation, atopic dermatitis alleviation, wound healing, and wound healing promotion.

## Description

### Technical Field

The present disclosure relates to a composition for improving skin condition including *Camellia japonica* callus-derived exosomes as an active ingredient.

### Background Art

Aging occurs throughout all organs of the body, and the skin likewise undergoes progressive aging with advancing years. Among the various manifestations of skin aging, wrinkles are the most common and prominent feature and serve as a key indicator of the degree of skin aging. Recent improvements in living standards along with growing interest in skin care have led to heightened attention toward skin aging. Although the aging process itself cannot be completely halted, various attempts have been devoted to delaying and mitigating skin aging and its representative symptom of wrinkle formation.

Skin aging can be divided into intrinsic and extrinsic aging depending on the causative factors involved. Intrinsic skin aging is primarily attributed to the loss of extracellular matrix (ECM) within the dermis, which consists of collagen, elastin, fibronectin, and proteoglycans. Aging of the dermal layer results in impaired ECM function, leading to thinning and flattening of the skin, wrinkle formation, reduced elasticity, skin atrophy, and dryness. Additionally, the aging process profoundly compromises skin appendages such as hair follicles, sweat glands, and sebaceous glands, causing a decline in their physiological functions and consequently delaying wound healing in response to external stimuli.

Among the extrinsic factors contributing to skin aging, ultraviolet (UV) radiation has been reported to accelerate skin aging and induce skin damage by stimulating melanogenesis following UV exposure. Additionally, free radicals generated through UV irradiation and cellular respiration play a critical role in damaging skin cells and tissues. Excessive free radical production disrupts the body's antioxidant defense system, resulting in inflammation, erythema, burns, immunosuppression, and DNA damage, ultimately accelerating the skin aging process. Furthermore, both intrinsic and extrinsic stimuli reduce the water content of the stratum corneum and decrease the levels of natural moisturizing factors (NMFs), leading to diminished skin hydration and increased skin dryness. These changes can ultimately compromise the skin barrier. As skin homeostasis becomes disrupted, the physical and chemical properties of the skin are altered, resulting not only in wrinkle formation, hyperpigmentation, moisture loss, and skin barrier dysfunction, but also in increased inflammation and impaired cellular regeneration capacity.

Therefore, effective antioxidant defense systems capable of suppressing and eliminating excessive free radicals both within the body and in the skin are essential for delaying and preventing skin aging. Consequently, interest in bioactive materials for skin improvement has steadily increased, and extensive research has focused on natural products for skin barrier protection and anti-aging applications. Although the commercial demand for natural products is rising, sustainable plant harvesting is inherently challenging, necessitating alternative stable production methods. In this regard, plant tissue culture has emerged as a valuable technology for industrial-scale applications, as it enables stable and sustainable production independent of external factors such as geographic location and climate conditions. Due to these advantages, plant tissue culture has been widely utilized for large-scale propagation and the development of genetically transformed plants, leading to active research and development in the fields of cosmetics, nutraceuticals, and pharmaceuticals.

*Camellia japonica* is an evergreen shrub belongs to the genus Camellia of the family Theaceae. It is known that while there are over 100 species in the Camellia genus found throughout Southeast Asia, one species grows natively in the southern parts of Korea. Additionally, numerous horticultural cultivars with diverse flower shapes, colors, and leaf characteristics have been developed and are widely cultivated as ornamental plants in Korea. It grows up to 3 to 7 m, and is famously found in the mountains and fields of Jeju Island and the southern regions of Korea. The seeds of *Camellia japonica* contain approximately 9.1% to 11.5% saturated fatty acids and high levels of unsaturated fatty acids, including 85.6% to 89.4% oleic acid and 1.3% to 2.9% linoleic acid. Due to its high oleic acid content, camellia oil is classified as a non-drying oil and has traditionally been used as hair oil, lubricant oil for precision machinery, and edible oil. According to Compendium of Materia Medica (Ben Cao Gang Mu), powdered camellia flowers have also been used for the treatment of burn injuries. Phytochemical investigations, particularly those conducted in Japan, have revealed that the leaves, seeds, and flowers of *Camellia japonica* contain a variety of bioactive compounds, including camellin, pipecolic acid, eugenol, camelliagenins A and B, tubakisaponins, triterpenes, tannins, benzenoids, steroids, flavonoids, and phenylpropanoids. In Japan, dried flower buds of *Camellia japonica* have been used in folk medicine as hemostatic agents for the treatment of hematemesis and intestinal bleeding. The powdered red flowers were reportedly administered in combination with children's urine, ginger juice, and alcoholic beverages. Furthermore, various biological activities of *Camellia japonica* have been reported, including antiprotozoal and antispasmodic effects, inhibition of dental calculus formation, suppression of alcohol absorption, and skin-whitening effects.

In recent years, exosomes have been increasingly utilized across various fields, such as biotechnology, pharmaceuticals, and cosmetics. Exosomes are small membrane-bound extracellular vesicles secreted by a wide range of cells, including those derived from mammals, bacteria, and plants. As mediators of intercellular communication, exosomes are nanosized extracellular vesicles, typically ranging from 50 to 200 nm in diameter. Due to their nanoscale size, exosomes function as carriers for transporting membrane proteins that are insoluble in water. Membrane proteins are embedded within the phospholipid bilayer and possess hydrophobic characteristics, making their direct transport between cells through the blood stream inherently challenging. On the contrary, exosomes consist of a phospholipid bilayer membrane that enables the efficient transport of membrane proteins. By encapsulating their cargos within the lipid bilayer, exosomes protect them from degradation by environmental or enzymatic factors. Furthermore, exosomes facilitate cellular penetration and perform a variety of physiological and pathological functions, including immune regulation and intercellular signaling. The unique biological properties of exosomes have led to their widespread application in cosmetic formulations. Therefore, efficient extraction methods for exosomes have been actively explored.

Recent studies have been directed toward the diverse beneficial activities of plant-derived exosomes. Unlike animal cells, plant cells have both a plasma membrane and a cell wall. The extracellular space located between the cell wall and plasma membrane is referred to as the apoplast. In addition to serving as a pathway for the transport of water and nutrients, the apoplast acts as a site for the accumulation of numerous extracellular vesicles (EVs) or nanovesicles commonly known as exosomes. Similar to animal-derived exosomes, plant-derived exosomes contain nucleic acids (DNAs/RNAs), proteins (cytokines and growth factors), and other bioactive constituents. Notably, plant-derived exosomes also encapsulate diverse plant-specific secondary metabolites. Additionally, plants contain a wide variety of lipid species, and plant-derived exosomes generally exhibit greater lipid diversity and higher lipid content than animal-derived exosomes, which may contribute to their broad spectrum of biological activities. As naturally occurring and vegan-derived nanovesicles, plant-derived exosomes can be readily internalized by heterologous animal cells. The superior biocompatibility of plant-derived exosomes compared to synthetic carriers has prompted extensive research into their application as key bioactive materials.

Human cell-derived extracellular vesicles face several challenges, including restricted functionality, as well as excessive costs and the need for specialized facilities for large-scale production. Additionally, their production commonly relies on animal-derived culture supplements, such as fetal bovine serum (FBS), which may raise safety concerns. On the contrary, plant-derived exosomes are free of safety concerns associated with animal-derived materials. Therefore, once their biological activities and mechanisms of action are fully elucidated, plant-derived exosomes are expected to serve as valuable ingredients for applications in functional foods, pharmaceuticals, and cosmetic products.

The use of diverse plant extracts from callus for the production of useful substances such as exosomes has attracted growing research interests. The related technologies disclosed in several patents in Korea, including Korean Patent Application Publication No. 10-2023-0144973, entitled "Method for manufacturing exosome derived from plant and plant-derived exosome produced by the method", Korean Patent No. 10-2461314, entitled "Composition for improving skin condition comprising exosome derived from Malus domestica fruit callus", and Korean Patent Application Publication No. 10-2023-0063541, entitled "Method for producing nanoexosome derived from Aloe bark callus and composition for skin improvement comprising nanoexosomes derived from Aloe bark callus prepared accordingly".

However, to date, no studies have yet reported cosmetic or pharmaceutical compositions that utilize exosomes derived from callus of *Camellia japonica* to simultaneously provide anti-inflammatory, antioxidant, whitening, wrinkle-reducing, hydrating, skin-regenerating, wound-healing, skin barrier-improving, atopic dermatitis-alleviating, and skin-soothing effects.

Accordingly, the present inventors investigated the biological activities of exosomes derived from callus of *Camellia japonica* and found that these exosomes effectively inhibited nitric oxide (NO) production, UV-induced free radical generation, extracellular melanin synthesis, matrix metalloproteinase-1 (MMP-1) expression, thymus and activation-regulated chemokine (TARC) production, and vascular endothelial growth factor (VEGF) expression, while enhancing aquaporin-3 (AQP3) and filaggrin expression and reducing intercellular gap formation. These biological activities collectively contribute to their anti-inflammatory, antioxidant, whitening, wrinkle-reducing, hydrating, skin-regenerating, wound-healing, skin barrier-improving, atopic dermatitis-alleviating, and skin-soothing effects. Based on the above findings, the present inventors completed the present disclosure.

### Disclosure

### Technical Problem

One objective of the present disclosure is to provide a composition for improving skin condition including *Camellia japonica* callus-derived exosomes as an active ingredient.

Another objective of the present disclosure is to provide a cosmetic composition for anti-inflammation, antioxidation, whitening, wrinkle reduction, hydration, skin regeneration, skin barrier improvement, atopic dermatitis alleviation, and skin soothing or a pharmaceutical composition for anti-inflammation, atopic dermatitis alleviation, wound healing, and wound healing promotion, each including *Camellia japonica* callus-derived exosomes as an active ingredient.

### Technical Solution

In order to accomplish the above objectives, the present disclosure provides a cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient.

In the present disclosure, the *Camellia japonica* callus-derived exosomes may be isolated and purified from Camellia japonica-derived callus or a conditioned medium thereof.

In the present disclosure, the callus may be induced from any one part selected from the group consisting of flowers, leaves, stems, roots, seeds, and fruits of *Camellia japonica*.

In the present disclosure, the *Camellia japonica* callus-derived exosomes may be isolated from at least one selected from the group consisting of callus-derived exosomes, a callus lysate, and a callus extract.

In the present disclosure, the exosomes may be extracellular vesicles having an average diameter of 50 to 200 nm.

In the present disclosure, the composition may be any one selected from the group consisting of a shampoo, a soap, a rinse, a surfactant-containing cleanser, a skin lotion, a toner, a cream, a lotion, an emulsion, a serum, an essence, a suspension, an emulsion, an ointment, a tonic, a treatment, a conditioner, a paste, a gel, an oil, a wax, a spray, an aerosol, a mist, and a powder.

The present disclosure further provides a pharmaceutical composition for anti-inflammation, atopic dermatitis alleviation, wound healing, and wound healing promotion including *Camellia japonica* callus-derived exosomes as an active ingredient.

In the present disclosure, the composition may be administered or treated by injection, microneedling, iontophoresis, application, or a combination thereof.

In the present disclosure, the composition may be any one selected from the group consisting of a liquid formulation, a patch formulation, an injectable formulation, an infusion formulation, and a spray formulation.

### Advantageous Effects

*Camellia japonica* callus-derived exosomes provided by the present disclosure effectively inhibit nitric oxide (NO) production, UV-induced free radical generation, extracellular melanin production, matrix metalloproteinase-1 (MMP-1) gene expression, thymus and activation-regulated chemokine (TARC) production, and vascular endothelial growth factor (VEGF) gene expression, while enhancing aquaporin-3 (AQP3) and filaggrin expression and reducing intercellular gap formation. These biological activities collectively contribute to their anti-inflammatory, antioxidant, whitening, wrinkle-reducing, hydrating, skin-regenerating, wound-healing, skin barrier-improving, atopic dermatitis-alleviating, and skin-soothing effects. Therefore, the exosomes can be advantageously used in cosmetic compositions for anti-inflammation, antioxidation, whitening, wrinkle reduction, hydration, skin regeneration, skin barrier improvement, atopic dermatitis alleviation, and skin soothing or in pharmaceutical compositions for anti-inflammation, atopic dermatitis alleviation, wound healing, and wound healing promotion.

### Description of Drawings

FIG. 1 illustrates the process of callus induction from *Camellia japonica* and mass propagation of callus through suspension culture.
FIG. 2 illustrates particle size and concentration of exosomes of Examples 1-1 to 1-3, Examples 2 and 3, and Comparative Example 1, as measured using a nanoparticle tracking analyzer (NTA, Particle Metrix).
FIG. 3 illustrates transmission electron microscopy (TEM) images of exosomes of Examples 1-1 to 1-3 and Comparative Example 1. FIG. 4 illustrates cell viability of mouse macrophages (RAW 264.7), mouse melanomas (B16F10), human keratinocytes (HaCaT), and human fibroblasts (CCD-986sk) treated with Examples 1-1 to 1-3 and Comparative Example 1.
FIG. 5 illustrates cell viability of mouse macrophages (RAW 264.7), mouse melanomas (B16F10), human keratinocytes (HaCaT), and human fibroblasts (CCD-986sk) treated with Examples 2 and 3 and Example 1-3.
FIG. 6 illustrates nitric oxide (NO) production in mouse macrophages (RAW 264.7) to evaluate anti-inflammatory effects of Examples 1-1 to 1-3 and Comparative Example 1.
FIG. 7 illustrates nitric oxide (NO) production in macrophages (RAW 264.7) to evaluate anti-inflammatory effects of Examples 2 and 3 and Example 1-3.
FIG. 8 illustrates the evaluation results of intracellular antioxidant effects of Examples 1-1 to 1-3 and Comparative Example 1 in human keratinocytes (HaCaT).
FIG. 9 illustrates melanin production in mouse melanomas (B16F10) to evaluate whitening effects of Examples 1-1 to 1-3 and Comparative Example 1.
FIG. 10 illustrates melanin production in mouse melanomas (B16F10) to evaluate whitening effects of Examples 2 and 3 and Example 1-3.
FIG. 11 illustrates matrix metalloproteinase-1 (MMP-1) mRNA expression in human fibroblasts (CCD-986sk) to evaluate wrinkle-reducing effects of Examples 1-1 to 1-3 and Comparative Example 1.
FIG. 12 illustrates matrix metalloproteinase-1 (MMP-1) mRNA expression in human fibroblasts (CCD-986sk) to evaluate wrinkle-reducing effects of Examples 2 and 3 and Example 1-3.

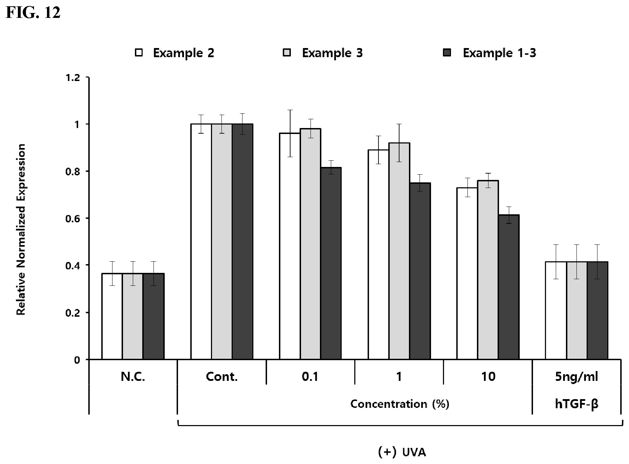

FIG. 12 illustrates aquaporin-3 (AQP3) mRNA expression in human keratinocytes (HaCaT) to evaluate hydrating effects of Examples 1-1 to 1-3 and Comparative Example 1.

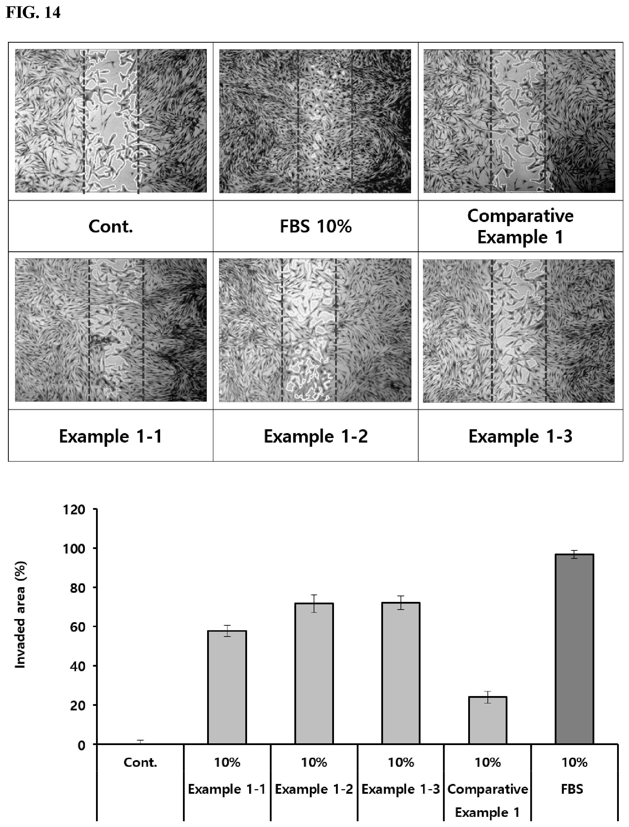

FIG. 14 illustrates wound healing assay results of human fibroblasts (CCD-986sk) treated with Examples 1-1 to 1-3 and Comparative Example 1.

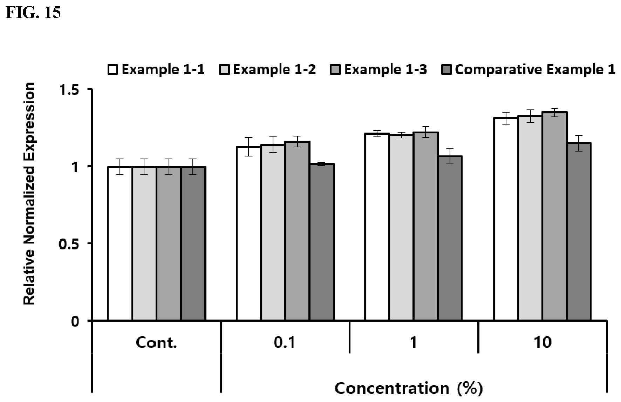

FIG. 15 illustrates filaggrin (FLG) mRNA expression in human keratinocytes (HaCaT) to evaluate skin barrier-improving effects of Examples 1-1 to 1-3 and Comparative Example 1.

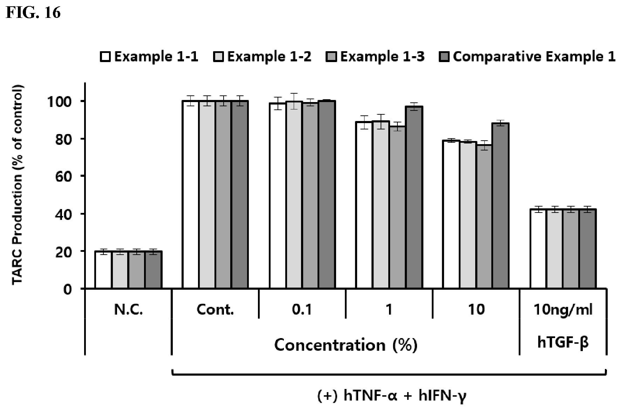

FIG. 16 illustrates thymus and activation-regulated chemokine (TARC) production in human keratinocytes (HaCaT) to evaluate atopic dermatitis-alleviating effects of Examples 1-1 to 1-3 and Comparative Example 1.

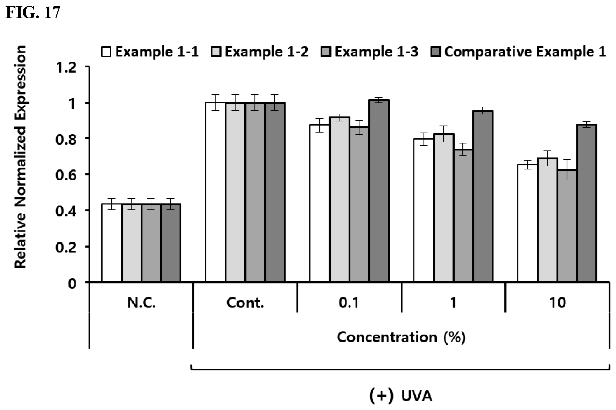

FIG. 17 illustrates vascular endothelial growth factor (VEGF) gene expression in human keratinocytes (HaCaT) to evaluate skin irritation-alleviating effects of Examples 1-1 to 1-3 and Comparative Example 1.

### Best Mode

Unless otherwise defined, all terms including technical and scientific terms used herein have the same meaning as commonly understood by one of ordinary skill in the art to which this disclosure belongs. Generally, the nomenclature used herein is well known in the art and is the nomenclature normally used.

As used herein, when any part "includes" or "contains" any element, this indicates that other elements are not excluded but may be further included unless otherwise specifically mentioned.

The term "callus" as used herein refers to an unorganized mass of plant parenchyma cells that is typically composed of undifferentiated cells. Callus can be induced from various plant organs, including flowers, leaves, stems, roots, seeds, and fruits. Callus induction and propagation are generally performed using methods publicly known in the field. More specifically, callus induction and propagation may be achieved by culturing plant tissues or explants on an appropriate tissue culture medium.

The term "exosomes" as used herein refers to nanosized membrane-bound vesicles secreted or released from plant cells or plant stem cells into the extracellular space. In the present disclosure, the exosomes are preferably extracellular vesicles (EVs) with an average particle size ranging from approximately 50 to 200 nm.

The term "extracellular vesicles (EVs)" as used herein collectively refers to membrane-bound vesicles secreted by cells for intercellular communication. EVs include exosomes, microvesicles, apoptotic bodies, and the like.

Therefore, according to one embodiment of the present disclosure, the present disclosure relates to a cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient.

According to one embodiment of the present disclosure, the *Camellia japonica* callus-derived exosomes may be isolated and purified from *Camellia japonica*-derived callus or a conditioned medium thereof.

The callus may be induced from any one part selected from the group consisting of flowers, leaves, stems, roots, seeds, and fruits of *Camellia japonica*.

According to one embodiment of the present disclosure, the exosomes may be isolated from at least one selected from the group consisting of callus-derived exosomes, a callus lysate, and a callus extract.

Various sources of plant-derived exosomes have been reported, including exosomes derived from plant callus, exosomes derived from plant juices, and exosomes derived from plant homogenates. Among these, plant callus-derived exosomes are employed in the present disclosure as an active ingredient. Comparative evaluation of exosomes obtained from plant juices, plant homogenates, and plant callus revealed that extracellularly released exosomes isolated via plant callus culture exhibited superior biological activity attributed to the preservation of key bioactive components originating from plant cells.

According to one embodiment of the present disclosure, the exosomes may be extracellular vesicles having an average diameter of 50 to 200 nm.

According to one embodiment of the present disclosure, the composition may be any one selected from the group consisting of a shampoo, a soap, a rinse, a surfactant-containing cleanser, a skin lotion, a toner, a cream, a lotion, an emulsion, a serum, an essence, a suspension, an emulsion, an ointment, a tonic, a treatment, a conditioner, a paste, a gel, an oil, a wax, a spray, an aerosol, a mist, and a powder. Among these, a lotion or cream is preferred.

In the cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient, the *Camellia japonica* callus-derived exosomes may be present in the composition at a concentration ranging from 1.0 × 10⁸ to 1.0 × 10¹² particles/mL, preferably from 2.0 × 10⁹ to 2.0 × 10¹⁰ particles/mL. However, the concentration is not limited thereto, provided that it is effective to provide the desired biological activities of the *Camellia japonica* callus-derived exosomes.

The cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient may further include ingredients commonly used in cosmetic compositions. Examples thereof include a moisturizer, an antioxidant, an oily component, a UV absorber, an emulsion, a surfactant, a thickener, an alcohol, a powder component, a colorant, an aqueous component, water, and various skin nutrients. These ingredients may be appropriately mixed within a range that does not impair the effects of the present disclosure.

The cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient may further include, in addition to the *Camellia japonica* callus-derived exosomes, commonly used skin-improving agents and/or moisturizing agents. These agents may be appropriately mixed within a range that does not impair the biological activity (e.g., anti-inflammatory, antioxidant, whitening, wrinkle-reducing, hydrating, skin-regenerating, skin barrier-improving, atopic dermatitis-alleviating, skin-soothing activities, and the like) of the exosomes.

The cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient may be applied to various dosage forms. Examples thereof include a patch, a mask pack, a mask sheet, a cream, a tonic, an ointment, a suspension, an emulsion, a paste, a lotion, a gel, an oil, a pack, a spray, an aerosol, a mist, a foundation, a powder, and an oil-control paper.

The cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient may be used for anti-inflammatory, antioxidant, whitening, wrinkle-reducing, hydrating, skin-regenerating, skin barrier-improving, atopic dermatitis-alleviating, and skin-soothing purposes, and may be prepared into any formulation commonly used in the field. Examples thereof include, but are not limited to, a patch, a mask pack, a mask sheet, a softening toner, a nourishing toner, an astringent toner, a nourishing cream, a massage cream, an eye cream, a cleansing cream, an essence, an eye essence, a cleansing lotion, a cleansing foam, a cleansing water, a sunscreen, a lipstick, a soap, a shampoo, a surfactant-containing cleanser, a bath preparation, a body lotion, a body cream, a body oil, a body essence, a body cleanser, a hair dye, and a hair tonic.

The cosmetic composition for anti-inflammation, antioxidation, whitening, wrinkle reduction, hydration, skin regeneration, skin barrier improvement, atopic dermatitis alleviation, and skin soothing including *Camellia japonica* callus-derived exosomes as an active ingredient may further include ingredients commonly used in cosmetic compositions. Examples thereof include conventional adjuvants such as an antioxidant, a stabilizer, a solubilizer, a vitamin, a pigment, and an aromatic; and a carrier. Additionally, in each formulation of the cosmetic composition, other ingredients may be appropriately selected and incorporated by those skilled in the art according to the type and intended use of the cosmetic composition.

As described above, the *Camellia japonica* callus-derived exosomes effectively inhibit nitric oxide (NO) production, UV-induced free radical generation, extracellular melanin production, matrix metalloproteinase-1 (MMP-1) gene expression, thymus and activation-regulated chemokine (TARC) production, and vascular endothelial growth factor (VEGF) gene expression, while enhancing aquaporin-3 (AQP3) and filaggrin expression and reducing intercellular gap formation. These biological activities collectively contribute to their anti-inflammatory, antioxidant, whitening, wrinkle-reducing, hydrating, skin-regenerating, wound-healing, skin barrier-improving, atopic dermatitis-alleviating, and skin-soothing effects. Therefore, the exosomes may be advantageously used in cosmetic compositions for anti-inflammation, antioxidation, whitening, wrinkle reduction, hydration, skin regeneration, skin barrier improvement, atopic dermatitis alleviation, and skin soothing or in pharmaceutical compositions for anti-inflammation, atopic dermatitis alleviation, wound healing, and wound healing promotion. Furthermore, the exosomes have a lower possibility of containing impurities compared to crude extracts or conditioned medium.

The present disclosure further relates to a pharmaceutical composition for anti-inflammation, atopic dermatitis alleviation, wound healing, and wound healing promotion including *Camellia japonica* callus-derived exosomes as an active ingredient.

According to one embodiment of the present disclosure, the composition may be administered or treated by injection, microneedling, iontophoresis, application, or a combination thereof.

According to one embodiment of the present disclosure, the composition may be any one selected from the group consisting of a liquid formulation, a patch formulation, an injectable formulation, an infusion formulation, and a spray formulation.

When used as a pharmaceutical composition for anti-inflammation, atopic dermatitis alleviation, wound healing, and wound healing promotion, the composition may further include a pharmaceutically acceptable carrier, excipient, or diluent. Examples of the carrier, excipient, and diluent include, but are not limited to, lactose, dextrose, trehalose, sucrose, sorbitol, mannitol, xylitol, erythritol, maltitol, starch, gum acacia, alginate, gelatin, calcium phosphate, calcium carbonate, calcium silicate, cellulose, methylcellulose, microcrystalline cellulose, polyvinylpyrrolidone, water, methyl hydroxybenzoate, propyl hydroxybenzoate, talc, magnesium stearate, and mineral oil. Additionally, the effective amount of the pharmaceutical composition according to the embodiment of the present disclosure refers to an amount sufficient for administration in order to achieve the desired anti-inflammatory, atopic dermatitis-alleviating, wound-healing, and wound healing-promoting effects.

Accordingly, the *Camellia japonica* callus-derived exosomes provided by the present disclosure effectively inhibit nitric oxide (NO) production, UV-induced free radical generation, extracellular melanin production, matrix metalloproteinase-1 (MMP-1) gene expression, thymus and activation-regulated chemokine (TARC) production, and vascular endothelial growth factor (VEGF) gene expression, while enhancing aquaporin-3 (AQP3) and filaggrin expression and reducing intercellular gap formation. These biological activities collectively contribute to their anti-inflammatory, antioxidant, whitening, wrinkle-reducing, hydrating, skin-regenerating, wound-healing, skin barrier-improving, atopic dermatitis-alleviating, and skin-soothing effects. Therefore, the exosomes may be advantageously used in cosmetic compositions for anti-inflammation, antioxidation, whitening, wrinkle reduction, hydration, skin regeneration, skin barrier improvement, atopic dermatitis alleviation, and skin soothing or in pharmaceutical compositions for anti-inflammation, atopic dermatitis alleviation, wound healing, and wound healing promotion.

### Mode for Invention

Hereinafter, the present disclosure will be described in more detail by way of Examples. These examples are provided only to aid understanding of the present disclosure, and it will be apparent to those skilled in the art that the scope of the present disclosure is not limited by these examples.

### <Example 1> In Vitro Introduction of Camellia japonica and Callus Induction

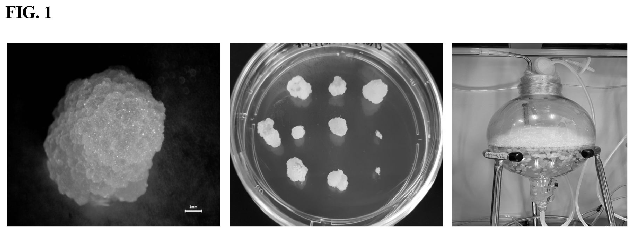

Leaves of *Camellia japonica* used in the present disclosure were collected in October from Yeosu, Jeollanamdo, Korea. The collected leaves were sterilized by immersion in 95% ethanol for 60 seconds, followed by treatment with a disinfectant solution (50% sodium hypochlorite + 0.1% Tween-20) for 20 minutes. The sterilized leaves were subsequently washed at least three times with sterile distilled water. The washed leaf tissues were cut into explants having a size of 2 × 2 mm using a scalpel. The callus induction efficiency was evaluated using combinations of plant growth regulators, including 2,4-dichlorophenoxyacetic acid (2,4-D, Sigma-Aldrich) and 6-benzylaminopurine (6-BA, Sigma-Aldrich). When the effects of different plant growth regulator combinations on callus induction rate and growth rate were compared, a medium containing 0.3 mg/L 2,4-dichlorophenoxyacetic acid and 1.0 mg/L 6-benzylaminopurine exhibited substantially higher callus induction and growth rates than a comparative medium containing 2.0 mg/L 2,4-dichlorophenoxyacetic acid, an auxin-type hormone, and 0.2 mg/L kinetin, a cytokinin-type hormone. These results indicate that the type and concentration of plant growth regulators employed in the present disclosure are optimally suitable for rapid callus induction and growth. Table 1 below shows the callus induction and growth rates of *Camellia japonica* depending on the type of growth regulators. Callus was induced on a basal MS medium containing 3% sucrose as a carbon source and 0.4% Gelrite as a gelling agent under dark culture conditions at 25 ± 2°C and 60% relative humidity. The callus induction status was monitored under a stereomicroscope (Zoom Stereo Microscope Head & Dual Light, KS-306). The induced callus was subcultured on a solid medium every 4 weeks. Through two to three rounds of subculture, elite cell lines were selected. The callus was subsequently transferred to a liquid MS medium containing 0.3 mg/L 2,4-dichlorophenoxyacetic acid (2,4-D, Sigma-Aldrich) and 1.0 mg/L 6-benzylaminopurine solution (6-BA, Sigma-Aldrich) and suspension-cultured in a 10-L bioreactor at 25 ± 2°C with an aeration rate of 0.2 vvm for four weeks (FIG. 1).

**Table 1**

| | Basal MS | Sucrose (%) | Cytokinin (mg/L) | | Auxin (mg/L) | ^{a}Callus induction rate (%) | ^{b}Callus growth rate |
|---|---|---|---|---|---|---|---|
| | | | 6-BA | Kinetin | 2,4-D | | |
| A | 1X | 3 | 1.0 | 0.0 | 0.3 | 98.3 | +++ |
| B | 1X | 3 | 0.0 | 0.2 | 2.0 | 33.3 | + |

^{a}Callus induction rate was defined as: callus induction rate (%) = (number of explants produced callus/total number of explants cultured)× 100.

^{b}The callus growth rate was recorded per day basis of callus induction and graded by comparison in three categories as very good (+++), average (++), bad (+).

### <Example 1-1> Isolation of Exosomes from Camellia japonica Leaf-Derived Callus

Following large-scale cultivation as in Example 1, a *Camellia japonica* leaf-derived callus culture was harvested and separated into callus and a conditioned medium by filtration, after which only the callus was collected. The collected callus was mixed with sterile distilled water at a ratio of 20 wt% and 80 wt%, respectively, followed by homogenization to obtain a homogenate. The resulting homogenate was subjected to centrifugation to remove impurities. Specifically, the homogenate was centrifuged at 1,000 to 2,000× g for 10 minutes and subsequently at 3,000 to 4,000× g for 10 to 20 minutes. After each centrifugation step, only the supernatant was collected while the pellet fraction was discarded. The supernatant was further centrifuged at 10,000 to 15,000× g for 30 to 60 minutes using an ultracentrifuge. The supernatant was filtered to remove residual materials and further improve its purity. The clarified supernatant was centrifuged at 100,000 to 150,000× g for 2 to 4 hours using an ultracentrifuge, allowing exosomes present therein to settle into the pellet. The resulting exosome-containing pellet was collected and resuspended in sterile distilled water to disperse the exosomes. Finally, the exosome suspension was sterilized through a 0.2 µm syringe filter to obtain *Camellia japonica* callus-derived exosomes.

### <Example 1-2> Isolation of Exosomes from Camellia japonica Leaf-Derived Callus Conditioned Medium

Following large-scale cultivation as in Example 1, a *Camellia japonica* leaf-derived callus culture was harvested and separated into callus and a conditioned medium by filtration, after which only the conditioned medium was collected. Exosomes were isolated from the collected conditioned medium in the same manner as in Example 1-1.

### <Example 1-3> Isolation of Exosomes from Mixture of Camellia japonica Leaf-Derived Callus and Conditioned Medium

Following large-scale cultivation as in Example 1, a *Camellia japonica* leaf-derived callus culture was harvested and separated into callus and a conditioned medium by filtration. The collected callus and conditioned medium were mixed at a ratio of 20 wt% and 80 wt%, respectively, followed by homogenization to obtain a homogenate. Exosomes were isolated from the homogenate in the same manner as in Example 1-1.

### <Example 2> Isolation of Exosomes from Camellia japonica Flower-Derived Callus

For the induction of callus, flowers of *Camellia japonica* were introduced into in vitro culture according to the same procedure as in Example 1. The callus was subsequently cultured in a 10-L bioreactor. Following large-scale cultivation, a *Camellia japonica* flower-derived callus culture was harvested and separated into callus and a conditioned medium by filtration. The collected callus and conditioned medium were mixed at a ratio of 20 wt% and 80 wt%, respectively, followed by homogenization to obtain a homogenate. Exosomes were isolated from the homogenate in the same manner as in Example 1-1.

### <Example 3> Isolation of Exosomes from Camellia japonica Seed-Derived Callus

For the induction of callus, seeds of *Camellia japonica* were introduced into in vitro culture according to the same procedure as in Example 1. The callus was subsequently cultured in a 10-L bioreactor. Following large-scale cultivation, a *Camellia japonica* seed-derived callus culture was harvested and separated into callus and a conditioned medium. The collected callus and conditioned medium were mixed at a ratio of 20 wt% and 80 wt%, respectively, followed by homogenization to obtain a homogenate. Exosomes were isolated from the homogenate in the same manner as in Example 1-1.

### <Comparative Example 1> Isolation of Exosomes from Camellia japonica Leaves

Sterilized and washed *Camellia japonica* leaves prepared as in Example 1 were mixed with sterile distilled water at a ratio of 20 wt% and 80 wt%, respectively, followed by homogenization to obtain a homogenate. Exosomes were isolated from the homogenate in the same manner as in Example 1-1.

### <Experimental Example 1> Characterization of Exosome Particles

For quantitative and physical characterization of exosomes isolated in Examples 1-1 to 1-3, Examples 2 and 3, and Comparative Example 1, analysis was performed using a nanoparticle tracking analyzer (NTA, Particle Metrix). The NTA used for measuring the particle concentration and particle size of exosomes was a Zetaview Zeta Potential model manufactured by Particle Metrix. Prior to analysis, exosome suspensions were diluted with sterile distilled water at an appropriate ratio. Measurements were carried out using a laser wavelength of 488 nm and an electrical conductivity of 1.00 µS/cm.

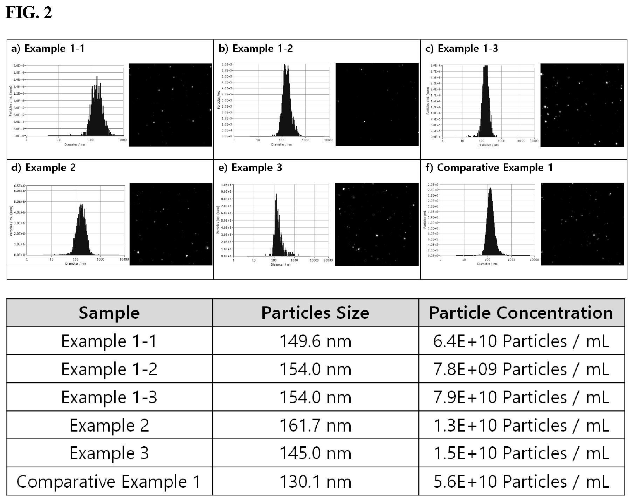

As a result, as shown in FIG. 2, particle concentration and particle size distribution in exosome samples were determined. The SPAN value indicated the uniformity of the samples, and the samples with a low span value demonstrated high purity. Based on the measured particle concentration, all exosome samples were normalized to a concentration of 2.0 × 10⁹ particles/mL and used as a 100% stock solution for experiments.

Meanwhile, to further examine morphology, the exosomes from Examples 1-1 to 1-3 and Comparative Example 1 were analyzed using a transmission electron microscope (JEOL, JEM-1010). Specifically, 10 µL of each exosome sample was deposited onto a carbon-coated copper grid and allowed to adsorb for 1 to 5 minutes at room temperature. For negative staining, the samples were treated with 2% uranyl acetate for 1 to 2 minutes. The stained exosomes were subsequently visualized using a transmission electron microscope.

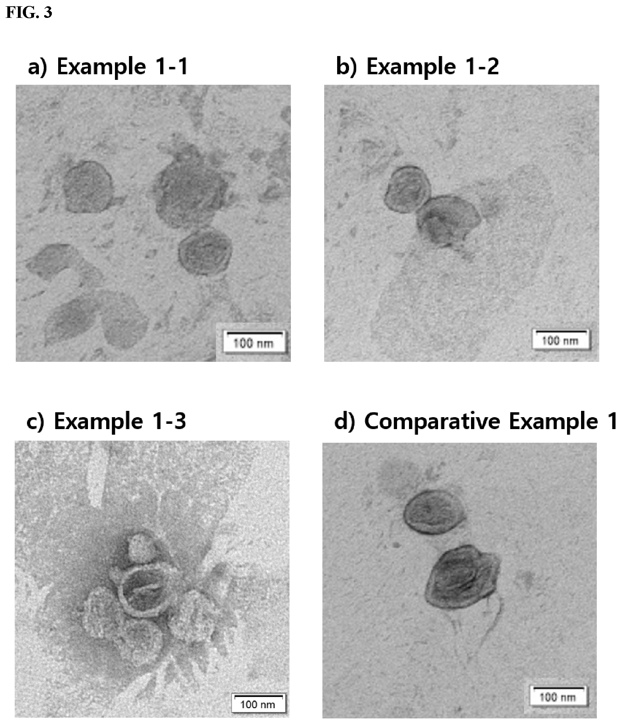

As a result, as shown in FIG. 3, the exosomes exhibited a spherical morphology surrounded by a phospholipid bilayer membrane, confirming the characteristic structural features of exosomes.

### <Experimental Example 2> Evaluation of Cytotoxicity: WST-1 assay

For in vitro efficacy evaluation, exosomes isolated in Examples 1-1 to 1-3, Examples 2 and 3, and Comparative Example 1 were administered at varying concentrations to determine whether they exhibited cytotoxicity toward each cell line.

Mouse macrophages (RAW 264.7), mouse melanomas (B16F10), human keratinocytes (HaCaT), and human fibroblasts (CCD-986sk) were used for a WST-1 assay. RAW 264.RAW 264.7, B16F10, and HaCaT cells were cultured in a Dulbecco's Modified Eagle Medium (DMEM) containing 10% fetal bovine serum (FBS) and 1% penicillin/streptomycin (P/S), while CCD-986sk cells were cultured in an Iscove's Modified Dulbecco's Medium (IMDM) containing 10% FBS and 1% P/S. All cells were maintained in an incubator with 37°C and 5% CO₂. The cells were seeded into 96-well plates at appropriate densities and cultured in an incubator with 37°C and 5% CO₂ to allow cell attachment to the plate. After 24 hours, the culture medium was then replaced with a fresh medium containing Examples 1-1 to 1-3, Examples 2 and 3, and Comparative Example 1 diluted at concentrations ranging from 0% to 10%. After 24 hours of treatment, the medium was removed and 10% WST-1 reagent was added to each well. After incubation for 2 hours in an incubator, absorbance was measured at 450 nm using an ELISA reader (BioTek, USA). Cell viability was calculated according to Equation 1 below:
[Equation 1] 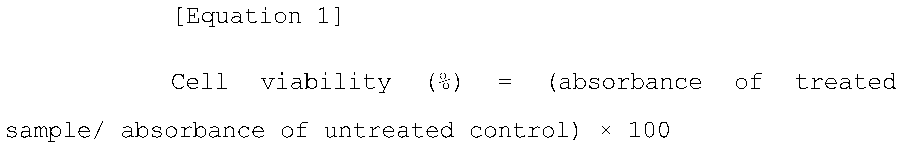 Cell viability (%) = (absorbance of treated sample/ absorbance of untreated control) × 100

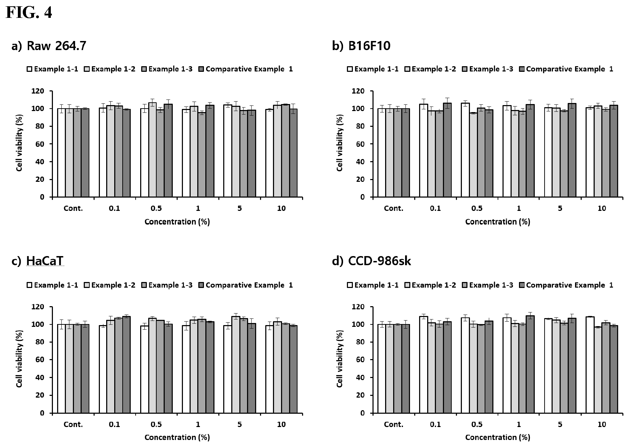

As a result, as shown in FIG. 4, FIGS. 4a to 4d presents the results of cytotoxicity evaluation in mouse macrophages (RAW 264.7), mouse melanomas (B16F10), human keratinocytes (HaCaT), and human fibroblasts (CCD-986sk), respectively. Examples 1-1 to 1-3 and Comparative Example 1 exhibited no significant cytotoxicity toward RAW 264.7, B16F10, HaCaT, or CCD-986sk cells at any tested concentration.

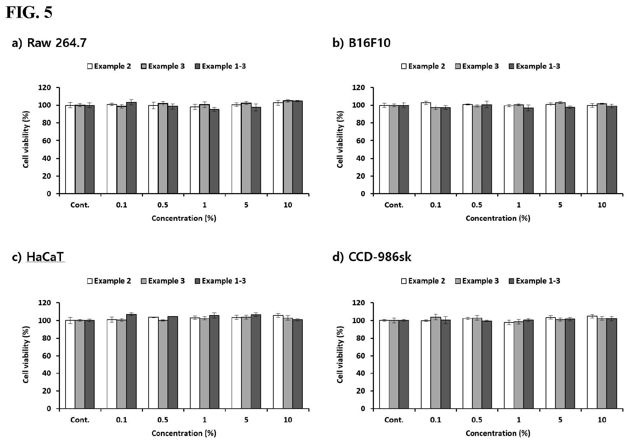

Furthermore, as shown in FIG. 5, Example 1-3, Example 2, and Example 3, exosomes isolated from callus cultures derived from different parts of *Camellia japonica*, also exhibited no detectable cytotoxicity toward RAW 264.7, B16F10, HaCaT, and CCD-986sk cells at any tested concentration.

### <Experimental Example 3> Test for Anti-Inflammatory Activity: Inhibition of Nitric Oxide Production

In order to evaluate the anti-inflammatory effects of Examples 1-1 to 1-3, Examples 2 and 3, and Comparative Example 1, nitric oxide (NO) production, which is an indicator of inflammatory responses, was measured using mouse macrophage (RAW 264.7) cells.

RAW 264.7 cells were seeded into a 24-well plate at a density of 2 × 10⁵ cells/well and cultured in an incubator with 37°C and 5% CO₂ for 24 hours to allow cell attachment. Exosome samples were diluted in a DMEM medium containing 1 µg/mL lipopolysaccharide (LPS), which is an inflammatory inducer that promotes NO production, at varying concentrations. As a positive control, N-Monomethyl-L-arginine acetate salt (L-NMMA) was prepared at a concentration of 50 µM. Following removal of the conditioned medium, the cells were treated with the diluted samples of Examples 1-1 to 1-3, Examples 2 and 3, Comparative Example 1 and the positive control and cultured for an additional 24 hours in an incubator with 37°C and 5% CO₂. Upon completion of incubation, the conditioned medium was collected from each well and transferred to a 96-well plate at 100 µL per well. Subsequently, 50 µL of Griess reagent A (naphthylethylenediamine buffer) and 50 µL of Griess reagent B (sulfanilamide buffer) were added to each well to obtain a final reaction volume of 200 µL. The mixture was incubated for 10 minutes, and absorbance was measured at 540 nm. Nitric oxide (NO) production was quantified using a standard curve generated from serially diluted nitrite standards.

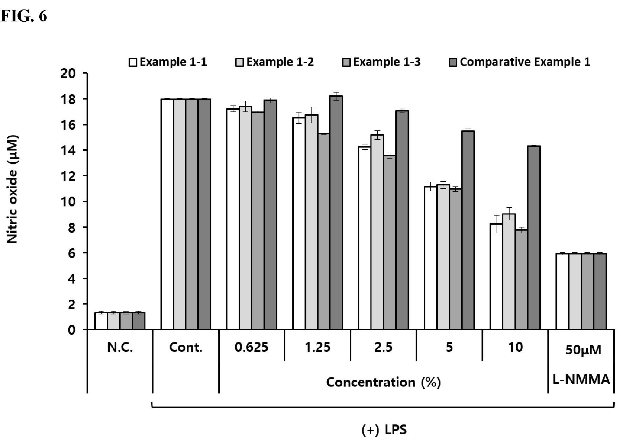

As a result, as shown in FIG. 6, Examples 1-1 to 1-3 and Comparative Example 1 were found to reduce NO production. Notably, Examples 1-1 to 1-3 exhibited superior NO production-inhibitory activity compared to Comparative Example 1. At a treatment concentration of 10%, NO production reduced by 54.19%, 49.85%, 56.73%, and 20.27% for Examples 1-1, 1-2, 1-3, and Comparative Example 1, respectively.

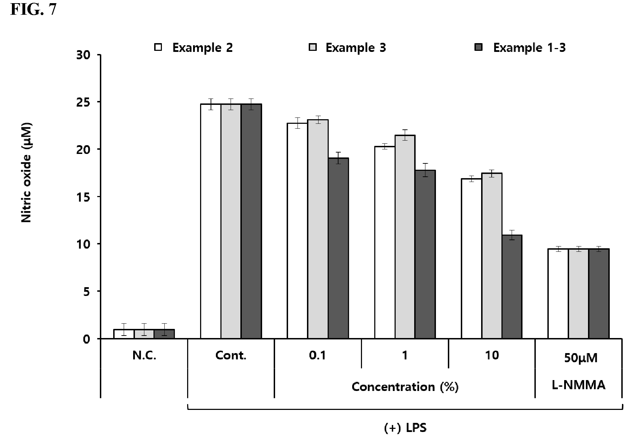

Furthermore, as shown in FIG. 7, the effects of Example 1-3, Example 2, and Example 3, exosomes isolated from callus cultures derived from different parts of *Camellia japonica*, on NO production were evaluated. At a treatment concentration of 10%, Example 1-3, Example 2, and Example 3 reduced NO production by 55.90%, 31.83%, and 29.52%, respectively. Among these, Example 1-3 exhibited the most pronounced inhibitory effect on NO production.

### <Experimental Example 4> Test for Antioxidant Activity: Intracellular Reactive Oxygen Species (ROS) Reduction Assay

In order to evaluate the antioxidant effects of Examples 1-1 to 1-3 and Comparative Example 1, intracellular antioxidant activity was assessed in human keratinocyte HaCaT cells using an H₂DCFDA assay.

HaCaT cells were seeded into a 96-well plate at a density of 1 × 10⁴ cells/well and cultured for 24 hours in an incubator with 37°C and 5% CO₂ to allow cell attachment. The culture medium was then replaced with a serum-free medium, and the cells were cultured for an additional 24 hours. After incubation with 5 mM CM-H₂DCFDA at 37°C for 30 minutes, the cells were treated with exosome samples of Examples 1-1 to 1-3 and Comparative Example 1 diluted at appropriate concentrations and 2 µM Trolox as a positive control andcultured for 1 hour in an incubator with 37°C and 5% CO₂. Subsequently, ROS generation was induced by UVB irradiation at 500 mJ/cm², and the cells were cultured for an additional 1 hour in an incubator with 37°C and 5% CO₂. After completion of the incubation, fluorescence intensity was measured at an excitation wavelength of 488 nm and an emission wavelength of 528 nm. Antioxidant activity was evaluated according to Equation 2 below:
[Equation 2] 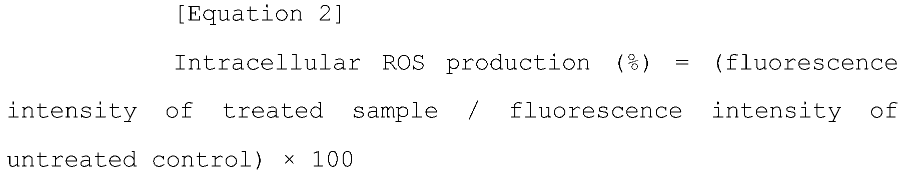 Intracellular ROS production (%) = (fluorescence intensity of treated sample / fluorescence intensity of untreated control) × 100

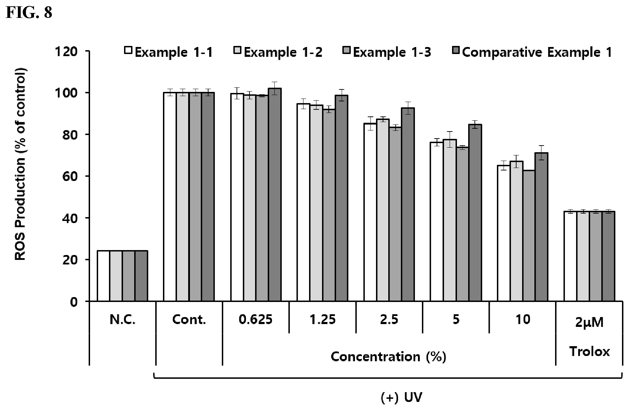

As a result, as shown in FIG. 8, Examples 1-1 to 1-3 and Comparative Example 1 were found to reduce UV-induced intracellular ROS production. Notably, Examples 1-1 to 1-3 exhibited superior ROS-reducing effects compared to Comparative Example 1. At a treatment concentration of 10%, ROS production reduced by 34.87%, 33.03%, 37.33%, and 28.80% for Examples 1-1, 1-2, 1-3, and Comparative Example 1, respectively.

### <Experimental Example 5> Test for Whitening Activity: Analysis of Extracellular Melanin Content

In order to evaluate the whitening effects of Examples 1-1 to 1-3, Examples 2 and 3, and Comparative Example 1, extracellular melanin content was measured using mouse melanoma (B16F10) cells.

B16F10 cells were seeded into a 24-well plate at a density of 3 × 10⁴ cells/well and cultured for 24 hours in an incubator with 37°C and 5% CO₂ to allow cell attachment. Exosome samples of Examples 1-1 to 1-3, Examples 2 and 3, and Comparative Example 1 were diluted in a phenol red-free DMEM containing α-melanocyte-stimulating hormone (α-MSH), which induces melanogenesis, at varying concentrations. As a positive control, β-arbutin was prepared at a concentration of 200 µg/mL. Following removal of the conditioned medium, the cells were treated with the diluted samples and the positive control and cultured for 48 to 72 hours in an incubator with 37°C and 5% CO₂. Upon completion of incubation, the conditioned medium was collected from each well, centrifuged, and transferred to a 96-well plate. Absorbance was measured at 490 nm, and extracellular melanin content was calculated according to Equation 3 below:
[Equation 3] 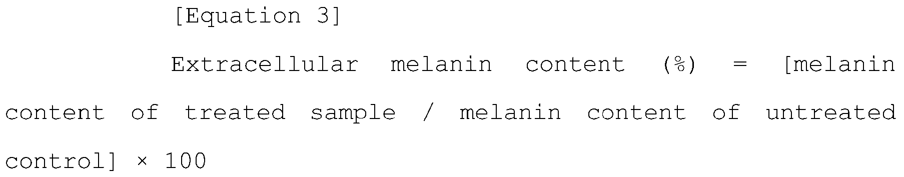 Extracellular melanin content (%) = [melanin content of treated sample / melanin content of untreated control] × 100

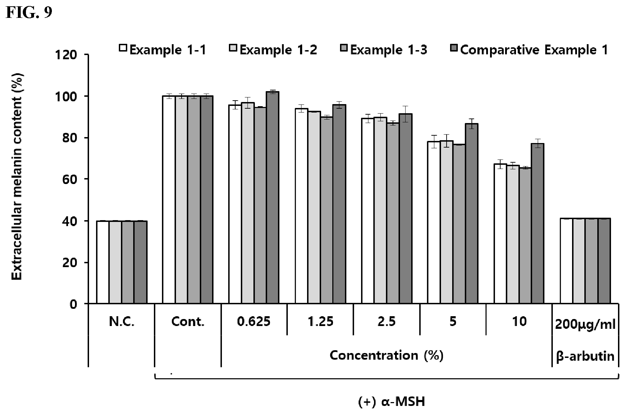

As a result, as shown in FIG. 9, Examples 1-1 to 1-3 and Comparative Example 1 were found to reduce extracellular melanin content. Notably, Examples 1-1 to 1-3 exhibited superior extracellular melanin-reducing effects compared to Comparative Example 1. At a treatment concentration of 10%, extracellular melanin content reduced by 32.81%, 33.60%, 34.60%, and 22.80% for Example 1-1, Example 1-2, Example 1-3, and Comparative Example 1, respectively.

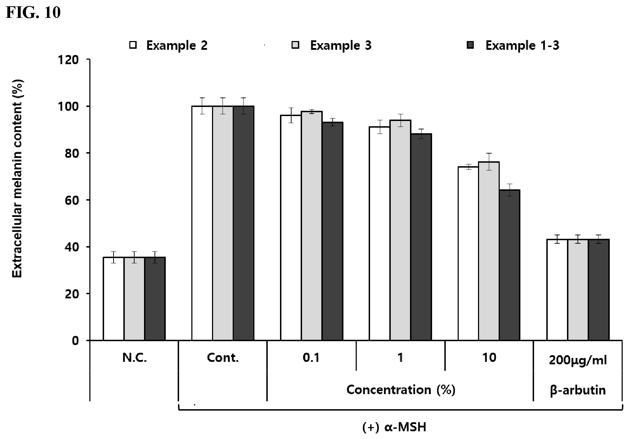

Furthermore, as shown in FIG. 10, the effects of Example 1-3, Example 2, and Example 3, exosomes isolated from callus cultures derived from different parts of *Camellia japonica*, on extracellular melanin content was evaluated. At a treatment concentration of 10%, Example 1-3, Example 2, and Example 3 reduced extracellular melanin content by 35.87%, 25.92%, and 23.82%, respectively. Among these, Example 1-3 exhibited the most pronounced inhibitory effect on extracellular melanin content.

### <Experimental Example 6> Test for Wrinkle-Reducing Activity: Measurement of MMP-1 mRNA Expression

In order to evaluate the wrinkle-reducing effects of Examples 1-1 to 1-3, Examples 2 and 3, and Comparative Example 1, the mRNA expression level of matrix metalloproteinase-1 (MMP-1), a collagen-degrading enzyme, was measured using human fibroblast cells.

CCD-986sk cells were seeded into a 24-well plate at a density of 2 × 10⁴ cells/well and cultured for 24 hours in an incubator with 37°C and 5% CO₂ to allow cell attachment. Following removal of the conditioned medium, the cells were washed with DPBS and exposed to UVA irradiation at a dose of 1 J/cm². Subsequently, the cells were treated with exosome samples of Examples 1-1 to 1-3, Examples 2 and 3, and Comparative Example 1 diluted at appropriate concentrations in a serum-free medium, followed by incubation. As a positive control, human transforming growth factor-β (hTGF-β) was prepared at a concentration of 5 ng/mL. To extract RNA from the cells treated with each sample, total RNA was isolated according to the protocol provided with a NucleoSpin RNA Kit (Macherey-Nagel). The extracted RNA was quantified at an absorbance of 260 nm using a NanoDrop instrument, and cDNA was synthesized from an appropriate amount of total RNA in an amplifier. The synthesized cDNA was mixed with target gene-specific primers and SYBR Green Supermix, a cyanine dye, and subjected to real-time polymerase chain reaction (real-time PCR) in a real-time PCR instrument to quantitatively evaluate target gene expression. The primer sequences and PCR conditions are summarized in Table 2 below. Relative gene expression levels was ultimately analyzed after normalization to the β-actin gene.

**Table 2**

| **Sequence No.** | **Gene** | **Sequence** | | **PCR conditions** |
|---|---|---|---|---|
| 1 | MMP-1 | Forward | 5' - CCCAGCGACTCTAGAAACAC - 3' | Initial polymerase activation at 95°C for 10 min, followed by 35 cycles of denaturation at 95°C for 15 s, annealing at 58°C for 30 s, and extension at 72°C for 30 s |
| 2 | | Reverse | 5' - GCCTCCCATCATTCTTCAGG - 3' | |
| 3 | β-actin | Forward | 5' - CATGAAGTGTGACGTGGACA - 3' | |
| 4 | | Reverse | 5' - CAGGGCAGTGATCTCCTTCT - 3' | |

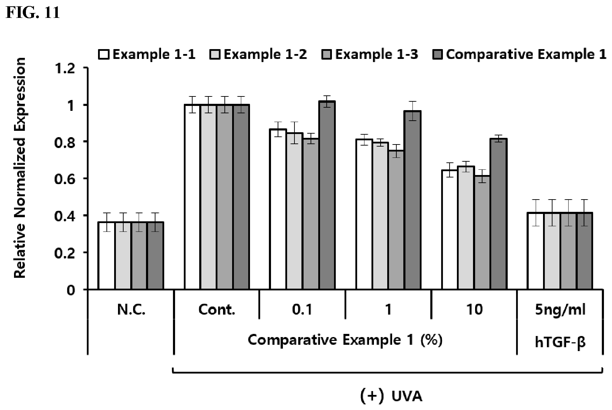

As result, as shown in FIG. 11, Examples 1-1 to 1-3 and Comparative Example 1 were found to reduce MMP-1 expression levels in a concentration-dependent manner. Notably, Examples 1-1 to 1-3 exhibited superior MMP-1 expression-inhibitory activity compared to Comparative Example 1. At a treatment concentration of 10%, MMP-1 expression reduced by 35.39%, 33.62%, 38.61%, and 18.36% for Example 1-1, Example 1-2, Example 1-3, and Comparative Example 1, respectively, relative to the control.

Furthermore, as shown in FIG. 12, the effects of exosomes isolated from callus cultures derived from different parts of *Camellia japonica* on MMP-1 mRNA expression were evaluated. At a treatment concentration of 10%, Example 1-3, Example 2, and Example 3 reduced MMP-1 mRNA expression by 38.61%, 27.00%, and 24.00%, respectively. Among these, Example 1-3 exhibited the most pronounced inhibitory effect on MMP-1 mRNA expression.

### <Experimental Example 7> Test for Hydrating Activity: Measurement of Aquaporin-3 (AQP3) mRNA Expression

In order to evaluate the hydrating effects of Examples 1-1 to 1-3 and Comparative Example 1, the expression level of aquaporin-3 (AQP3) mRNA was measured using human keratinocyte (HaCaT) cells.

HaCaT cells were seeded into a 24-well plate at a density of 1 × 10⁵ cells/well and cultured for 24 hours in an incubator with 37°C and 5% CO₂ to allow cell attachment. Subsequently, the cells were treated with exosome samples of Examples 1-1 to 1-3 and Comparative Example 1 diluted at appropriate concentrations in a serum-free medium, followed by incubation for an additional 24 hours. To extract RNA from the cells treated with each sample, total RNA was isolated according to the protocol provided with a NucleoSpin RNA Kit (Macherey-Nagel). The extracted RNA was quantified at an absorbance of 260 nm using a NanoDrop instrument, and cDNA was synthesized from an appropriate amount of total RNA in an amplifier. The synthesized cDNA was mixed with target gene-specific primers and SYBR Green Supermix, a cyanine dye, and subjected to real-time polymerase chain reaction (real-time PCR) in a real-time PCR instrument to quantitatively evaluate target gene expression. The primer sequences and PCR conditions are summarized in Table 3 below. Relative gene expression levels were ultimately analyzed after normalization to the β-actin gene.

**Table 3**

| **Sequence No.** | **Gene** | **Sequence** | | **PCR Conditions** |
|---|---|---|---|---|
| 5 | AQP3 | Forward | 5' - AGACAGCCCCTTCAGGATTT - 3' | Initial polymerase activation at 95°C for 10 min, followed by 35 cycles of denaturation at 95°C for 15 s, annealing at 58°C for 30 s, and extension at 72°C for 30 s |
| 6 | | Reverse | 5' - TCCCTTGCCCTGAATATCTG - 3' | |
| 7 | β-actin | Forward | 5' - CATGAAGTGTGACGTGGACA - 3' | |
| 8 | | Reverse | 5' - CAGGGCAGTGATCTCCTTCT - 3' | |

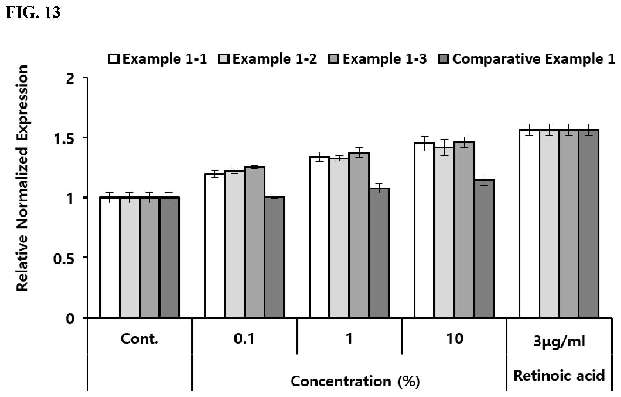

As a result, as shown in FIG. 13, Examples 1-1 to 1-3 and Comparative Example 1 were found to increase AQP3 expression levels in a concentration-dependent manner. Notably, Examples 1-1 to 1-3 exhibited superior stimulatory activity on AQP3 expression compared to Comparative Example 1. At a treatment concentration of 10%, AQP3 expression increased by 45.18%, 41.63%, 46.28%, and 14.87% for Example 1-1, Example 1-2, Example 1-3, and Comparative Example 1, respectively, relative to the control.

### <Experimental Example 8> Test for Skin-Regenerating and Wound-Healing Activities: Wound Healing assay

In order to evaluate the skin-regenerating and wound-healing effects of Examples 1-1 to 1-3 and Comparative Example 1, a wound healing assay was performed using human fibroblasts.

CCD-986sk cells were seeded into a 24-well plate provided with a wound healing insert at a density of 5 × 10⁴ cells/well and cultured for 24 hours in an incubator with 37°C and 5% CO₂. After confirming that the cells had grown to more than 80% confluence, the wound healing insert was removed and the wells were washed with DPBS. Subsequently, the cells were treated with exosome samples of Examples 1-1 to 1-3 and Comparative Example 1 diluted at appropriate concentrations in a serum-free medium, followed by incubation for an additional 48 hours. The extent to which an artificially generated intercellular gap was filled was observed under a microscope to evaluate cell migration, and invaded area was calculated according to Equation 4 below:
[Equation 4] 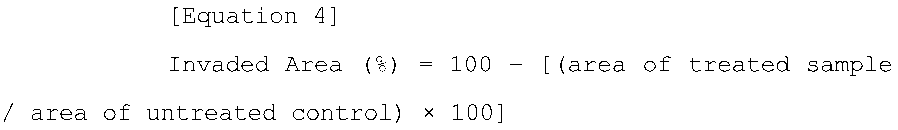 Invaded Area (%) = 100 - [(area of treated sample / area of untreated control) × 100]

As a result, as shown in FIG. 14, Examples 1-1 to 1-3 and Comparative Example 1 were found to promote narrowing of the intercellular gap. Notably, Examples 1-1 to 1-3 exhibited superior invaded area values compared to Comparative Example 1, thereby demonstrating their skin-regenerating and wound-healing activities. At a treatment concentration of 10%, the invaded area values increased by 57.67%, 71.81%, 72.22%, and 24.03% for Example 1-1, Example 1-2, Example 1-3, and Comparative Example 1, respectively, relative to the control.

### <Experimental Example 9> Test for Skin Barrier-Improving Activity: Measurement of Filaggrin (FLG) mRNA Expression

In order to evaluate the skin barrier-improving effects of Examples 1-1 to 1-3 and Comparative Example 1, the mRNA expression level of filaggrin (FLG) was measured using human keratinocyte (HaCaT) cells.

HaCaT cells were seeded into a 24-well plate at a density of 1 × 10⁵ cells/well and cultured for 24 hours in an incubator with 37°C and 5% CO₂ to allow cell attachment. Subsequently, the cells were treated with exosome samples of Examples 1-1 to 1-3 and Comparative Example 1 diluted at appropriate concentrations in a serum-free medium, followed by incubation for an additional 24 hours. To extract RNA from the cells treated with each sample, total RNA was isolated according to the protocol provided with a NucleoSpin RNA Kit (Macherey-Nagel). The extracted RNA was quantified at an absorbance of 260 nm using a NanoDrop instrument, and cDNA was synthesized from an appropriate amount of total RNA in an amplifier. The synthesized cDNA was mixed with target gene-specific primers and SYBR Green Supermix, a cyanine dye, and subjected to real-time polymerase chain reaction (real-time PCR) in a real-time PCR instrument to quantitatively evaluate target gene expression. The primer sequences and PCR conditions are summarized in Table 4 below. Relative gene expression levels were ultimately analyzed after normalization to the β-actin gene.

**Table 4**

| **Sequence No.** | **Gene** | **Sequence** | | **PCR Conditions** |
|---|---|---|---|---|
| 9 | FLG | Forward | 5'- GCTGAAGGAACTTCTGGAAAAG - 3' | Initial polymerase activation at 95°C for 10 min, followed by 35 cycles of denaturation at 95°C for 15 s, annealing at 58°C for 30 s, and extension at 72°C for 30 s |
| 10 | | Reverse | 5'- GCCAACTTGAATACCATCAGAAG - 3' | |
| 11 | β-actin | Forward | 5' - CATGAAGTGTGACGTGGACA - 3' | |
| 12 | | Reverse | 5' - CAGGGCAGTGATCTCCTTCT - 3' | |

As a result, as shown in FIG. 15, Examples 1-1 to 1-3 and Comparative Example 1 were found to increase FLG expression levels in a concentration-dependent manner. Notably, Examples 1-1 to 1-3 exhibited superior stimulatory activity on FLG expression compared to Comparative Example 1. At a treatment concentration of 10%, FLG expression increased by 31.59%, 32.89%, 35.35%, and 15.29% for Example 1-1, Example 1-2, Example 1-3, and Comparative Example 1, respectively, relative to the control.

### <Experimental Example 10> Alleviation of Atopic Dermatitis: Test for Inhibition of TARC Production

In order to evaluate the atopic dermatitis-alleviating effects of Examples 1-1 to 1-3 and Comparative Example 1, the production level of thymus and activation-regulated chemokine (TARC) was measured using human keratinocyte (HaCaT) cells.

HaCaT cells were seeded into a 24-well plate at a density of 1 × 10⁵ cells/well and cultured for 24 hours in an incubator with 37°C and 5% CO2 to allow cell attachment. Exosome samples of Examples 1-1 to 1-3 and Comparative Example 1 were diluted in a serum-free DMEM containing 10 ng/mL TNF-α and 10 ng/mL IFN-γ, which are inflammatory inducers, at varying concentrations. As a positive control, transforming growth factor-β (TGF-β) was prepared at a concentration of 10 ng/mL. Following removal of the conditioned medium, the cells were treated with the diluted samples of Examples 1-1 to 1-3, Examples 2 and 3, Comparative Example 1 and the positive control and cultured for 24 hours in an incubator with 37°C and 5% CO₂. Upon completion of incubation, the conditioned medium was collected, and TARC production was measured. For measurements, an ELISA assay was performed according to the protocol provided with an ELISA kit (BioLegend, 441104). The amount of TARC produced was quantified using a TARC standard curve obtained from the ELISA assay and expressed as a percentage relative to the control.

As a result, as shown in FIG. 16, Examples 1-1 to 1-3 and Comparative Example 1 were found to reduce TARC production. Notably, Examples 1-1 to 1-3 exhibited superior TARC production-inhibitory activity compared to Comparative Example 1. At a treatment concentration of 10%, TARC production reduced by 21.21%, 21.68%, 23.62%, and 11.82% for Example 1-1, Example 1-2, Example 1-3, and Comparative Example 1, respectively, relative to the control.

### <Experimental Example 11> Test for Skin-Soothing Activity: Measurement of VEGF mRNA Expression

In order to evaluate the skin-soothing effects of Examples 1-1 to 1-3 and Comparative Example 1, the mRNA expression level of vascular endothelial growth factor (VEGF) was measured using human keratinocyte (HaCaT) cells.

HaCaT cells were seeded into a 24-well plate at a density of 1 × 10⁵ cells/well and cultured for 24 hours in an incubator with 37°C and 5% CO₂ to allow cell attachment. To induce skin irritation, the cells were exposed to UVA irradiation at a dose of 0.5 J/cm². Subsequently, the cells were treated with exosome samples of Examples 1-1 to 1-3 and Comparative Example 1 diluted at appropriate concentrations in a serum-free medium, followed by incubation for 24 hours. To extract RNA from the cells treated with each sample, total RNA was isolated according to the protocol provided with a NucleoSpin RNA Kit (Macherey-Nagel). The extracted RNA was quantified at an absorbance of 260 nm using a NanoDrop instrument, and cDNA was synthesized from an appropriate amount of total RNA in an amplifier. The synthesized cDNA was mixed with target gene-specific primers and SYBR Green Supermix, a cyanine dye, and subjected to real-time polymerase chain reaction (real-time PCR) in a real-time PCR instrument to quantitatively evaluate target gene expression. The primer sequences and PCR conditions are summarized in Table 5 below. Relative gene expression levels were ultimately analyzed after normalization to the β-actin gene.

**Table 5**

| **Sequence No.** | **Gene** | **Sequence** | | **PCR Conditions** |
|---|---|---|---|---|
| 13 | VEGF | Forward | 5' - CATCCAATCGAGACCCTGGT - 3' | Initial polymerase activation at 95°C for 10 min, followed by 35 cycles of denaturation at 95°C for 15 s, annealing at 58°C for 30 s, and extension at 72°C for 30 s |
| 14 | | Reverse | 5' - ATCTCTCCTATGTGCTGGCC - 3' | |
| 15 | β-actin | Forward | 5' - CATGAAGTGTGACGTGGACA - 3' | |
| 16 | | Reverse | 5' - CAGGGCAGTGATCTCCTTCT - 3' | |

As a result, as shown in FIG. 17, Examples 1-1 to 1-3 and Comparative Example 1 were found to reduce VEGF mRNA expression levels in a concentration-dependent manner. Notably, Examples 1-1 to 1-3 exhibited superior VEGF mRNA expression-inhibitory activity compared to Comparative Example 1. At a treatment concentration of 10%, VEGF mRNA expression reduced by 34.62%, 31.16%, 37.44%, and 12.36% for Example 1-1, Example 1-2, Example 1-3, and Comparative Example 1, respectively, relative to the control.

Although the specific configurations of the present disclosure have been described in detail, those skilled in the art will appreciate that this description is provided as preferred embodiments and should not be construed as limiting the scope of the present disclosure. Therefore, the substantial scope of the present disclosure is defined by the appended claims and equivalents thereof.

### Industrial Applicability

C*amellia japonica* callus-derived exosomes provided by the present disclosure exhibit excellent antiinflammatory, antioxidant, whitening, wrinkle-reducing, hydrating, skin-regenerating, wound-healing, skin barrier-improving, atopic dermatitis-alleviating, and skin-soothing activities. Therefore, the exosomes can be advantageously used in cosmetic compositions for anti-inflammation, antioxidation, whitening, wrinkle reduction, hydration, skin regeneration, skin barrier improvement, atopic dermatitis alleviation, and skin soothing or in pharmaceutical compositions for anti-inflammation, atopic dermatitis alleviation, wound healing, and wound healing promotion.

## Claims

1. A cosmetic composition for anti-inflammation, antioxidation, whitening, wrinkle reduction, hydration, skin regeneration, skin barrier improvement, atopic dermatitis alleviation, and skin soothing comprising *Camellia japonica* callus-derived exosomes as an active ingredient.

2. The cosmetic composition of claim 1, wherein the *Camellia japonica* callus-derived exosomes are isolated and purified from *Camellia japonica*-derived callus or a conditioned medium thereof.

3. The cosmetic composition of claim 2, wherein the callus is induced from any one part selected from the group consisting of flowers, leaves, stems, roots, seeds, and fruits of *Camellia japonica*.

4. The cosmetic composition of claim 1, wherein the *Camellia japonica* callus-derived exosomes are isolated from at least one selected from the group consisting of callus-derived exosomes, a callus lysate, and a callus extract.

5. The cosmetic composition of claim 1, wherein the exosomes are extracellular vesicles having an average diameter of 50 to 200 nm.

6. The cosmetic composition of claim 1, wherein the composition is any one selected from the group consisting of a shampoo, a soap, a rinse, a surfactant-containing cleanser, a skin lotion, a toner, a cream, a lotion, an emulsion, a serum, an essence, a suspension, an emulsion, an ointment, a tonic, a treatment, a conditioner, a paste, a gel, an oil, a wax, a spray, an aerosol, a mist, and a powder.

7. A pharmaceutical composition for anti-inflammation, atopic dermatitis alleviation, wound healing, and wound healing promotion comprising *Camellia japonica* callus-derived exosomes as an active ingredient.

8. The pharmaceutical composition of claim 7, wherein the composition is administered or treated by injection, microneedling, iontophoresis, application, or a combination thereof.

9. The pharmaceutical composition of claim 7, wherein the composition is any one selected from the group consisting of a liquid formulation, a patch formulation, an injectable formulation, an infusion formulation, and a spray formulation.
